(19) **Europäisches Patentamt**
**European Patent Office**
**Office européen des brevets**

(11) **EP 4 464 297 A1**

(12) # EUROPEAN PATENT APPLICATION

(43) Date of publication:
**20.11.2024 Bulletin 2024/47**

(21) Application number: **24176213.7**

(22) Date of filing: **16.05.2024**

(51) International Patent Classification (IPC):
***A61H 1/02*** *(2006.01)*

(52) Cooperative Patent Classification (CPC):
**A61H 1/0274; A61H 1/0237;** A61B 5/389;
A61H 2201/1207; A61H 2201/1638;
A61H 2201/1642; A61H 2201/165;
A61H 2201/1659; A61H 2201/5061;
A61H 2201/5064; A61H 2230/605

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.05.2023 EP 23174131**

(71) Applicants:
  • **Vrije Universiteit Brussel**
    **1050 Brussel (BE)**
  • **Imec VZW**
    **3001 Leuven (BE)**

(72) Inventors:
  • **LANGLOIS, Kevin**
    **1030 Schaarbeek (BE)**
  • **DE WINTER, Joris**
    **2610 Wilrijk (BE)**
  • **VANDERBORGHT, Bram**
    **1560 Hoeilaart (BE)**
  • **VERSTRATEN, Tom**
    **1050 Brussel (BE)**

(74) Representative: **Winger**
    **Mouterij 16 bus 101**
    **3190 Boortmeerbeek (BE)**

(54) **REHABILITATION ROBOT OR EXOSKELETON AND COMPONENTS THEREOF**

(57) The present invention relates to a physical interface device for providing a physical interface between a user and rigid components of a machine, e.g. a cuff. The physical interface device comprises a soft component for providing a soft contact to the user, and one or more sensors which are integrated in the physical interface device, wherein at least one of the sensors is configured for sensing a force inside the physical interface device. The present invention also relates to a machine comprising a physical interface device.

FIG. 2

**Description**

**Technical field of the invention**

The present invention relates to machines interacting with users, such as rehabilitation robots for patients or exoskeleton robots, as well as to components thereof. More particularly, the present invention relates to a physical interface device for providing contact between a user and rigid components of a machine or robot, as well as machines comprising such a physical interface device.

**Background of the invention**

Traditionally, mobilization therapy is performed manually by therapists. This is physically demanding since a leg for example, weighs 15% of total body mass and needs to be mobilized for 30 min straight. Additionally, therapists have to take care of many patients at a time, this is up to 16 per day. That means 30min per person without any break which makes it difficult to provide intensive care to all patients. As a consequence, we are currently not providing optimal care.

Rehabilitation robots have been developed as a way to improve treatment outcomes and reduce the workload of therapists while also reducing healthcare costs. These robots can provide long-term, multi-modal rehabilitation training. By way of illustration, the state of the art is discussed with focus on upper-limb rehabilitation robots. There are two main types of upper limb rehabilitation robots : (i) End-effector robots, such as Amadeo from Tyromotion or Luna EMG from EGZOTech, which are systems with only one attachment to the human body, where no alignment between patient and robot joint is required and (ii) Exoskeleton robots, such as Harmony SHR™ from Harmonics Robotics and Armeo Power from Hocoma, are systems with multiple connections to the human body. The joints between the connecting links of the robot align with the joints of the human.

Exoskeletons have the advantages that the multiple joints can be individually controlled, allow training of specific muscles and allow for force/torque measurements on each body connection, including the estimation of specific human joint torques, but require longer setup times, since it needs to be configured to the specific patient as the joint of the exoskeleton has to be aligned with the joint of the patient. Furthermore, they have a more complex structure and a customized design for a specific limb, are unsafe when they are badly calibrated to the patient and are difficult to carry and transport.

End-effectors on the other hand have a simple structure, are easier to carry or transport, provide solutions that are on average cheaper, mostly because of the simple structures and provide for a faster setup, resulting in less time wasted for the physiotherapist. In some cases, the patients can even strap themselves. Typical disadvantages are that they are mostly only capable of doing simple motions, do not provide solutions for functional motion training and operate in such a way that force generated at a distal interface changes the positions of other joints simultaneously, making isolated movement of single joints difficult.

Both end-effector and exoskeleton device rely on at least one attachment (i.e. physical interface) to the human body. These attachments have problems regarding safety and comfortability, as reported in literature, hindering mainstay practical use of rehabilitation robots in healthcare. Soft-tissue related adverse events are the most recurring events, preventing the medical field to provide long-lasting and safe therapy. The challenge relates to the pressure distributed on the skin tissues. When attaching a robot to a human body, excessive pressure can lead to skin injuries. A related problem is power transmission between machine and human body. Researchers have observed critical power losses (up to 50%) at the physical interface level, leading to reduced benefits of wearing the devices.

Moreover, inadequate controllability has limited adoption of rehabilitation devices, since it directly limits the range of operations we can assist. In essence, this boils down to the ability of the robot to capture the state and intention of the user. A popular biophysical sensor for intention-recognition is bipolar electromyography (EMG). This sensor can enable triggered assistance rehabilitation, allowing the patient to initiate a movement without any assistance. The robot will only start to assist the patient after some performance variables have reached a threshold. This encourages the patient to self-initiate a movement, which is believed to be beneficial in motor learning.

Drawbacks of current robotic rehabilitation devices include:

1. Complexity of programming motions: With current devices, programming a motion is difficult for therapists. It requires extensive training and devices become obsolete.
2. Lack of movement: Existing machines are limited in their range of motion and in their modes of motion (limited to planar motions, limited to analytical motions).
3. Lack of mobility: machines are heavy, bulky and often stationary.
4. Lack of versatility or personalization: devices are custom made for the limb they assist, or for the type of patient (such as bed-ridden, ambulatory, post-op). Different patients require different movements or exercises to train, hence high flexibility of the device is required. In order to cover all possible rehabilitation exercises with current technology, a therapist would require multiple devices.

5. Time to set-up makes machines obsolete. Placing a patient inside a rehabilitation robot usually requires careful alignment of anatomical joints with the robotic ones. This requires expertise and training to prevent dangerous situations. When a therapy consists of 30 min sessions, clinicians cannot afford to spend one third of that time setting up.

6. Safety: skin-related injuries are prevalent and are hindering prolonged use.

7. User intention: Current technologies do not allow intention-based rehabilitation. Intention-based rehabilitation can increase engagement, both cognitively and physically, and leads to improved outcomes.

Existing solutions often only focus on two dimensional planar motions and are not able to perform complex more functional motions (e.g. drinking, reaching tasks).

1. Complexity of operation:

a. Other solutions focus only on simple motions (i.e. motions in one plane).

b. Programming by demonstration is made easier by adding a big handle on the end-effector. As a result, it is easier to move the robot around. However, this still needs two hands to operate, leaving no possibility for the therapist to hold the patient. Holding the patient is critical for correct handling/manipulation.

2. Lack of movement

a. Exoskeletons solve this by trying to mimic as accurately as possible the skeleton of the patient. This requires addition of passive degrees of freedom (added complexity, bulk).

3. Lack of mobility

a. Have a small device that is easier to carry, but you will need to sacrifice a lot on level of complexity of the motion.

4. Lack of versatility or personalization:

a. No solutions available that can handle different body types (e.g. both leg and arm).

b. Exoskeletons require a lot of tweaking to adjust to different patient. Moreover they are custom made for a specific body type. And often do not allow for the full set of exercises required for every patient (not enough degrees of freedom).

5. Time to set-up

a. End effector solutions keep the type of exercise simple in order to reduce the setup time (e.g. move in only one plane of motion). And have less degrees of freedom to move around in order to simplify the programming task of the therapist.

b. Extensive training.

6. Safety

a. Current solutions limit time of operation (sessions are limited) and provide additional padding in case of skin injuries (skin reddening, blisters).

7. User intention

a. Using gamification, the patient can be easier motivated, since they will have a feeling of influence during the therapy.

b. Using external forces, existing systems focus on assist as needed. This way the patient is only assisted in parts of the exercise when it is actually required. However, there is no way to tell if the patient starts slacking during the entire exercise.

There is, thus, still a need in the art for devices and methods that address at least some of the above problems.

## Summary of the invention

It is an object of embodiments of the present invention that a device is provided providing safety and/or comfortability when using a machine, e.g., a rehabilitation robot or exoskeleton.

It is an advantage of embodiments of the present invention that a physical interface device is provided, providing a sensorized interface which can be attached to the human body and can detect intentions to move, and quantify how much effort is being performed by the wearer during use of the machine, such as during performing tasks using the exoskeleton or during physical therapy.

The interface can be connected to a robotic arm, to further assist the person in achieving functional tasks, such as for example but not limited to brushing teeth, reaching for a glass of water, etc. This is especially useful for neurological patients who require repetitive and intense training to relearn these daily tasks.

By supplementing therapists with this data, the recovery process of the patient can be better monitored and evaluated, allowing a better personalization of therapy, e.g. rehabilitation therapy. By supporting therapists with the robotic device one can reduce their physical workload and allow them to treat more patients at the same time.

It is an advantage of at least some embodiments of the present invention that accurate intention of the patient is captured when using machines, e.g., rehabilitation robots or exoskeletons, so that the machine behaviour can adapt accordingly. It is for example an advantage of embodiments of the present invention that the intention of the patient is captured accurately so that a patient is not only triggering a motion assisted by the robot and "rides" out the rest of the movement but further provides effort during the rest of the movement, since slacking on the side of the patient may result in a decreased recovery.

It is an advantage of embodiments of the present invention that easy and fast setting up of a machine, e.g., rehabilitation robot or exoskeleton, is provided, since the physical interface device avoids the need for placement of separate sensors.

It is an advantage of embodiments of the present invention that due to the integration of the sensor or sensors inside the physical interface device, the positioning of the sensors with respect to the user is more accurate.

In a first aspect, the present invention relates to a physical interface device for providing a physical interface between a user and rigid components of a machine, e.g., a rehabilitation system or exoskeleton, e.g. the physical interface device also being referred to as a cuff, the physical interface device comprising

a soft component for providing a soft contact to the user, and
one or more sensors are integrated in or on the physical interface device, wherein at least one of the sensors is configured for sensing a force inside the physical interface device.

It is an advantage of embodiments of the present invention that a rehabilitation robot and/or corresponding robotic rehabilitation platform may be provided that can replicate the sense of touch of a physiotherapist.

It is an advantage of embodiments of the present invention that use of mechanical power in conjunction with close physical interaction with patients does not result in safety and comfort issues by using a particular physical interface device.

It is an advantage of at least embodiments of the present invention that the physical interface device can track the pressure exerted on the patient's skin, providing additional safety and comfort. It is an advantage of embodiments of the present invention that patients can receive more frequent therapy with higher intensity, particularly in the early mobilization phase.

The device may comprise an output or is configured to provide data to an output for indicating information regarding the application of the physical interface device to the user or regarding movement or movement intentions by the user, based on data of the one or more sensors integrated in the physical interface device, optionally in combination with data regarding external forces operating on the physical interface device.

It is an advantage of embodiments of the present invention that a physical interface device with integrated pressure sensors allows identifying how the physical interface device is applied to the user, e.g. when straps are tightened, and/or allows data-driven. When straps are tightened, a pressure increase can be registered.

It is an advantage of embodiments of the present invention that skin-related injuries can be avoided by taking into account the pressure that occurs between the physical interface device and the user.

It is an advantage of embodiments of the present invention that indication can be given that strapping pressure is sufficient, or safe, resulting in a decreased need of training to use the device, resulting in the possibility to use the device more easily and in a safer way.

It is an advantage of embodiments of the present invention that pressure sensors may allow for intention-detection, i.e. detection of the intention to perform a movement by the user.

It is an advantage of embodiments of the present invention that the system allows for detecting where the arm is placed inside the physical interface device, e.g. by determining the center of pressure.

One or more sensors may be configured so as to provide the ability to measure biological signals directly on the human skin.

The one or more sensors may comprise one or more force and/or pressure sensors.

In one set of embodiments, the one or more sensors comprise at least one force sensor, for example multiple force sensors. In some embodiments, forces may be sensed at multiple locations inside the physical interface device, e.g. cuff. The one or more force sensors may be adapted for measuring force in three dimensions, e.g. in three directions. The forces may be measured in an x, y and z direction. In some embodiments, the x, y and z direction may form a carthesian reference system.

The one or more force sensors may be part of or form a small tactile sensor. In some embodiments different small tactile sensors may be implemented at different positions. In some embodiments, the small tactile sensor may contain a plurality, e.g. 12 or more, such as 24 or more force sensor voxels, each voxel being a force sensor. Each of these force sensors may in some embodiments allow for measuring force in three directions, e.g. three dimensions. In this way, also shear force may be taken into account.

It is an advantage of embodiments of the present invention that novel features can be calculated, such as for example that an accurate centre of pressure (COP) can be determined. The latter may in some embodiments be based on multiple sensor cells.

According to some embodiments of the present invention, multiple force sensors may be positioned around the circumference inside of the physical interface device. The latter may for example allow for performing mechanomyography. To detect muscle deformation, forces typically may be measured at multiple locations. The latter may allow to distinguish between an increased force by muscle deformation or an increased force by e.g. increase of the external machine force or robot force.

It is an advantage of embodiments of the present invention that force peaks can be predicted, e.g. accurately predicted. When injuries occur in physical human machine interfaces or physical human robot interfaces, the injury is mostly located at these force peaks. Correctly detecting force peaks means one can make a safer physical interface.

It is an advantage of embodiments of the present invention that by implementing a plurality of sensors, one can more easily identify where the physical interface device is located with respect to the limb, thus allowing to better distinguish between the change of a muscle force or e.g. external forces.

The goal of the physical interface is to attach a machine to the human body. The tighter this connection the better the energy transmission. However, there is a trade-off since a tighter strapped cuff will also be less comfortable. The perfect level of strapping is a trade-off between comfort and energy transmission. The tightness will have an influence on the internal forces that are measured. The tighter the attachment the higher the internal forces, without any effect on the external forces. The latter may be calibrated.

Depending on the limb to which the physical interface device is to be attached to, the position of the sensors for measuring forces may be selected as function of the position where typically peak forces may occur, such as for example where the bone is closer to the skin.

The one or more force and/or pressure sensors may be configured for measuring a pressure distribution on skin tissues.

The one or more sensors may be configured for 3 dimensional tactile force sensing.

The one or more sensors may comprise one or more sensors for capturing the intention of the user for performing a certain movement.

The one or more sensors may comprise one or more electromyography (EMG) sensors.

The one or more sensors may combine at least one electromyography sensor, at least one pressure sensor and at least one inertial measurement sensor.

The physical interface device may comprise releasable connections for releasebly connecting the physical interface device to components, e.g. a robotic arm, of the machine, e.g., rehabilitation robot or exoskeleton.

It is an advantage of embodiments of the present invention that the machine, e.g., rehabilitation robot or exoskeleton, may be programmed by demonstration. Demonstration is an intuitive robot programming technique. Using the built in IMU in the sensorized interface, one can program the exercise by off-robot demonstration. The programming may for example be performed when the patient is wearing the cuff, but the cuff is not attached to the machine or robot. One then gets a trajectory from the UMI and one can recreate the desired exercise once the machine or robot is attached to the cuff. In this way, therapists only have the move the patient limb around (which is easier for them).

In one aspect, the present invention also relates to a machine, such as for example a rehabilitation robot for rehabilitation of a user or an exoskeleton, the machine comprising a physical interface device as described above. The machine, e.g. rehabilitation robot or exoskeleton, or the physical interface device may comprise one or more sensors for measuring the external force on the limb, e.g. through external forces on the physical interface device induced by the machine. In some examples the external forces may be induced by a robotic arm in the machine.

The machine, e.g. rehabilitation robot or exoskeleton, may combine internal forces measured with the one or more sensors integrated in/on the physical interface device with external forced induced on the physical interface device and combine these force measurements for deriving information regarding the application of the physical interface device to the user or regarding movement or movement intentions by the user and/or for deriving peak forces applied to the limb.

The machine may be an end-effector robot.

The machine may be a rehabilitation robot for upper-body extremities and/or lower-body extremities. The rehabilitation robot may be such that it can be adapted for rehabilitation of upper-body extremities, such as arms, and for rehabilitation of lower-body extremities, such as legs. Depending on the use, e.g. different physical interface devices may be used.

The machine may use a robotic arm as actuation device.

The robotic arm may have 6 spatial degrees of freedom and can be altered over time.

According to embodiments of the present invention, the rehabilitation robot thus may comprise a robotic arm having 7 degrees of freedom, 6 spatial degrees of freedom and time. It is an advantage of embodiments of the present invention that any kind of motion possible by the human arm can be achieved, resulting in the possibility for making functional exercises. It is an advantage of embodiments of the present invention that also complex motions can be induced giving the possibility to train specific muscles, when part of the body can be grounded, or such that resistive or assistive forces are provided depending on the requirements of the therapy.

The physical interface device or machine may comprise or may be configured to communicate with a user interface, for giving feedback to the therapist and/or to the patient.

The physical interface device or machine may be adapted for performing data analytics, including providing data or processing data, the data comprising one or more of pressure readings, EMG readings, robot peak force, robot current position and programmed reference trajectory, amount of transferred work between patient and robot, level of active participation, cuff strapping pressure and performance of patient (for example whether the patient/user reaches goals regarding the range of motion and/or the force.

Rehabilitation is a repetitive process that requires coordinated movements for effective treatment. However, patient compliance and motivation can be challenging due to the monotony, intensity, and cost of most therapy routines.

It is an advantage of at least some embodiments of the present invention that an intention-based rehabilitation approach that uses bio-signals, such as surface electromyographic sensors (sEMG) can be provided. It is an advantage of at least some embodiments of the present invention that performance can be more easily quantified than with state of the art techniques and that engagement can be stimulated and cost-effective treatment can be provided. It is an advantage of embodiments of the present invention that patient compliance and functional outcomes can be good, e.g. improved with respect to at least some of the state of the art techniques.

In one aspect, the present invention relates to an exoskeleton comprising a physical interface device according to embodiments of the first aspect of the present invention. Indeed, the present invention is not strictly limited to applications in rehabilitation but may be applied in any type of exoskeleton for supporting a user, e.g., during a physical activity. It is an advantage of embodiments of the present invention that good use of and good support by the exoskeleton may be achieved.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

**Brief description of the drawings**

FIG. 1 is an exemplary physical interface device in accordance with embodiments of the present invention.

FIG. 2 is a schematical representation of a SAFeR robot in accordance with embodiments of the present invention.

FIG. 3 is a block scheme of an experiment for rehabilitation for testing a physical interface device in accordance with embodiments of the present invention.

FIG. 4A and FIG. 4B are photographs of a SAFeR rehabilitation robot in use during said experiment.

FIG. 5 is a schematic representation of a second experimental session for testing a physical interface device in accordance with embodiments of the present invention.

FIG. 6 shows plots of experimental results, showing calibration results of the tested machine learning approaches.

The upper row represents the calibration plot of the models, e.g. the mean predicted probabilities (MPP) vs the fraction of positives (FOP). The lower row shows the histogram of the occurrences of the MPP.

FIG. 7 is a plot of an $\Delta$ALP estimated during 15 repetitions of an experiment for two different groups. The solid lines represent the mean value per each group and the shaded areas stand for the 95% confidence intervals.

FIG. 8 shows plots of said $\Delta$ALP along with three other performance indicators for an experiment for two different groups. The height of the bars represents the mean value of the distribution and the solid black lines represent the 95% confidence interval.

FIG. 9 shows plots of results on the level of engagement and perceived workload during an experiment, in particular, scores of the questionnaires, SAM and NASA-TLX, administered to the two experimental groups. The height of the bars represents the mean value of the distribution and the solid black lines represent the 95% confidence interval.

FIG. 10 shows an embodiment wherein shear force is used to detect relative motion between the arm and the physical interface device, according to an embodiment of the present invention.

## Description of illustrative embodiments

The present invention will be described with respect to particular embodiments and with reference to certain drawings and a particular exemplary illustrative embodiment, but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. The word "comprising" according to the invention therefore also includes as one embodiment that no further components are present. Thus, the scope of the expression "a device comprising means A and B" should not be interpreted as being limited to devices consisting only of components A and B. It means that with respect to the present invention, the only relevant components of the device are A and B.

Similarly, it is to be noticed that the term "coupled", also used in the claims, should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

In a first aspect, the present invention relates to a physical interface device for providing a physical interface between a user and rigid components of a machine, e.g., a rehabilitation system or an exoskeleton, e.g. a cuff, the physical interface device comprising

    a soft component for providing a soft contact to the user, and
    one or more sensors are integrated in the physical interface device.

The soft component may be a component that is more soft than the rigid components of the machine, e.g., rehabilitation system or exoskeleton. It may be e.g. foam based, although embodiments are not limited thereto. Further standard and optional components may be as set out in the summary and in the claims. Standard and optional features are also shown in the following drawing and description.

According to embodiments of the present invention the physical interface device is a physical Human Robot-Interface, what we call the 'cuff". It is the accessory to the device that allows to capture data about the patient that provides benefits. Specifically, the physical interface device may have in one embodiment integrated EMG electrodes, that measure muscular activity, and pressure sensors, to measure internal cuff pressures. Muscular activity can be used to detect intention of motion and estimate fatigue.

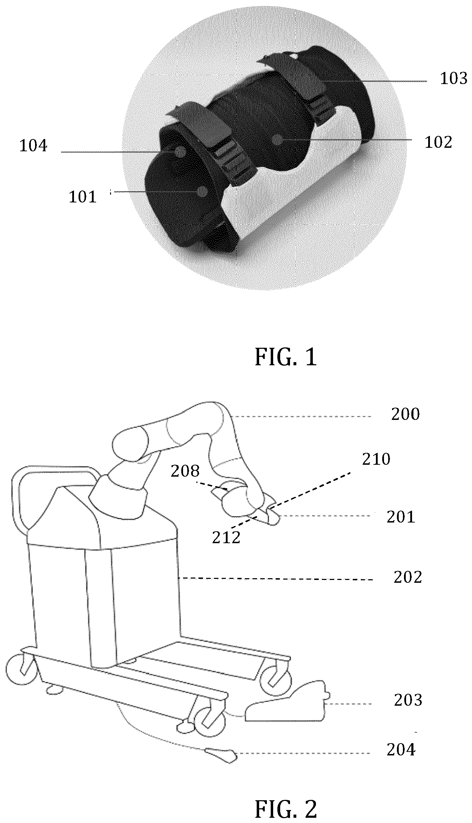

An image of an exemplary physical interface device is shown in FIG. 1.

According to embodiments, the physical interface device may provide for

- Estimation of level of participation of the patient based on sensor data (muscle activity, internal cuff pressures, robot measured forces, position)
- Strapping pressure measurement for estimation of caused skin injuries. A soft safety layer that feedbacks to the therapist if any pressure thresholds have been passed. No standards exist for the link between strapping pressure and injury. The system may provide feedback.
- Programming by demonstration technique when cuff is not attached to the machine or robot arm using IMU sensors. Using a quick tool changer interface between cuff and robot we are able to easily attach and detach the patient.
- Proxy based sliding mode controller for extra layer of safety (soft safety). The therapist will be able to set maximum forces the robot will exert on the patient. The robot will never go over the threshold. If this does happen, the system will go into a safety stop mode.

In some embodiments, when using force sensors in the interface, e.g. cuff, musclemyography can be performed. In other words, based on the deformation of the limb, the muscle activity can be measured. This is possible for muscles and limbs inside the cuff.

The physical interface device or the machine, e.g., rehabilitation robot or exoskeleton, may comprise or be configured to communicate with a user interface, for giving feedback to the therapist and/or to the patient. The machine or device or robot may be adapted for performing data analytics, although embodiments are not limited thereto. Data that may be used in the data analytics may include one or more of: pressure readings, EMG readings, robot peak force, robot current position and programmed reference trajectory, amount of transferred work between patient and robot, level of active participation, cuff strapping pressure and performance of patient (for example whether the patient/user reaches goals regarding the range of motion and/or the force).

As indicated above, the physical interface device may be combined with or part of a machine, e.g., rehabilitation robot or exoskeleton, operating like an end-effector. It is an advantage of such a machine, e.g., rehabilitation robot or exoskeleton, that one is not bound by the limits of kinematic alignment between the joints. This significantly reduces set-up time.

According to some embodiments, physical interface devices comprise a sensorized interface having 3 integrated sensor modalities: electromyography, pressure, inertial measurement unit. In some embodiments, EMG data can be fused with pressure to increase the accuracy of motion prediction. By measuring the pressure inside the cuff we can estimate muscle force, since the volume of the limb changes with exerted muscle force. Such data may for example be processed in a local processor, which may be part of the physical interface device, may be processed in a processor being part of the machine, e.g., rehabilitation robot or exoskeleton, or may be processed externally to the system. Combining the data of these sensors with the data measured by the machine or robot, such as for example with measured external forces, torques and positions, one can more accurately model the intention of the patient. The latter information may be used for increasing motivation by the patient.

In some embodiments, a strain sensor could be used, allowing to measure how much stretching or bending occurs, which also may allow for detecting pressure or force changes.

In a second aspect, the present invention relates to a rehabilitation robot for rehabilitation of a user, the rehabilitation robot comprising a physical interface device as described in the first aspect. Further standard and optional components may be as set out in the summary and in the claims. Standard and optional features are also shown in FIG. 2.

In one aspect, the present invention relates to an exoskeleton comprising a physical interface device according to embodiments of the first aspect of the present invention.

The present invention thus may also relate to a machine comprising a physical interface device according to embodiments of the first aspect of the present invention.

FIG. 2 shows the SAFeR rehabilitation robot or platform being composed of a robotic manipulator supported by a trolley, and a sensorized cuff which is attached to the robotic manipulator. Moreover, it uses a safety switch for the patient to pause the therapy session, and a pedal for the therapist to record motions.

The machine, e.g., rehabilitation robot, according to an exemplary embodiment of the present invention may comprise one or more of the following components -the example not being limited for the present invention):

- Trolley

    ∘ Which may have actuated feet to stabilize during execution of a therapy.
    ∘ Which may have 4 omnidirectional wheels so that it is easy to move around.
    ∘ A handle bar may be attached to the back to drive the device around.

- Sensorized Cuff

    ∘ Attached at the end of the robot, and used to attach the patient limb to the machine. It contains sensors to measure the state of the patient, as described in the first aspect.

- Cobot (currently an LBR Med, but the invention is not limited thereto).

    ∘ The robot may have at least 6DOF, since it is advantageous to assure that one can do any type of functional exercises. Currently a 7DOF robot arm is used, since the redundant joint ensures even more complex exercises are possible.
    ∘ The present example provides a cobot for handling upper body limbs. Since on average the arm is around 6-7% of the body weight, it may have a lift force of 10Kg (in order to handle all body types).
    ∘ The cobot may be mounted on the trolley under an angle, in order to

- Pedal

    ∘ Used to indicate start and end of a demonstration. In future versions this might change (e.g. when we start using the IMU off-robot demonstrations)

- Patient safety switch

    ∘ Button for the patient to start or pause the session.

- Broad procedure of a normal therapy session

    ∘ The robot will move to one side, depending on what side of the body is the focus of the rehabilitation (i.e. left/right arm or leg).

According to some embodiments :

The rehabilitation robot uses programming by demonstration.

The cobot of an exemplary embodiment of this invention may be composed of 7 joints and can carry loads of 14kg. These 7 joints mean that the workspace of the robot is large, and that many analytical motions can be performed as well as functional tasks. It is an advantage that the system may provide active assistance for functional tasks.

The robotic arm may be supported by a trolley. The trolley may be a cart with 4 rotative wheels, and it may possess an active lifting system. This means that electric actuators may lift the trolley to provide mechanical grounding. The platform can be moved around, similarly to a shopping cart.

SAFeR may be used to assist the legs, for example to provide mobilization in the post-op phase, as well as for the upper body. Specific cuffs may be provided for each body segment, which are easy to swap, and different sizes.

According to some embodiments, the system may comprise an insert used for attaching the sensors thereto, allowing an efficient installation and positioning of the sensors.

According to some embodiments, the system also may comprise a protection part for protecting the electronics in the system, e.g. made of any suitable material, such as e.g. plastics.

It is to be understood that although preferred embodiments, specific constructions and configurations, as well as materials, are discussed herein for devices according to the present invention, various changes or modifications in form and detail may be made without departing from the scope of this invention. For example, any formulas given above are merely representative of procedures that may be used. Steps may be added or deleted to methods described within the scope of the present invention.

In one example, the rehabilitation platform comprises a physical interface device used to attach the patient to the robot, being the part of the platform that makes it possible to mimic the physiotherapist sense of touch. The physical interface device is designed for safety and comfortability. By modeling human bodies, the physical interface device design can be shaped in such a way to better fit one or all patients. Additionally, using precise strapping mechanisms, patients can be more accurately strapped for increased comfort. Second, embedding sensors directly into the physical interface device results in easier and faster strapping of patients (reduced workload for physiotherapist results in reduced financial load). The sensors capture biological signals (i.e. internal cuff pressures and muscle activity) directly on the human body, and can be used as feedback for the physiotherapist to optimize the rehabilitation strategy. On the Data level, sensor data may be interpreted and visualized in an understandable way to the therapist. Soft-tissue adverse events during therapy (e.g. skin injury) can be counteracted by giving feedback about the strapping pressure distribution inside the cuff (integrated pressure sensors). Moreover, in order to increase outcome after rehabilitation, recognizing the intention of the patient by interpreting all data streams measured in the physical interface device and in the robot. The robot responsible for actuating the mobilization of the patient is a crucial element in the platform. Since the platform is in continuous contact with a patient, the first importance on the Robot level is the safety framework. Operation limits within which the robot can freely operate have been defined, outside of which the robot will go into a safety mode. For normal operation modes (i.e. mobilization of the patient), results have focused on the one hand on easy programming of new exercise motions (decrease the time a therapist has to setup the system). Programming is done using demonstrations, where the therapist records a motion by physically moving the robot and patient along the exercise trajectory. Afterwards, these exercises are replayed with different robot control modes that can be chosen by the physiotherapist. Early mobilization can rely on a passive controller, while later can be more relied on an active controller (using Proxy Based Sliding Mode Control) or adaptive controller (variable assistance based on level of motivation of the patient). Finally, all these achievements enabled the development of the robotic rehabilitation platform SAFeR, developed in the context of the Kuka Innovation Award challenge. The platform is partly evaluated on the Medical level. Testing has focused on evaluating the platform with healthy subjects. Moreover, functional testing has been performed, evaluating the platform in a hospital setting.

By way of illustration, embodiments of the present invention not being limited thereby, a further exemplary embodiment is discussed below, showing standard and optional elements and features of some embodiments of the present invention and showing advantages thereof.

Example: Experimental Validation of a User Active Level of Participation-Adapting Robot for Upper Limb Rehabilitation

Although below, a specific robot or machine for upper limb rehabilitation is described, the machine may be any machine, e.g., any type of rehabilitation robot or may be an exoskeleton.

Robotics is gaining more and more attention in the medical field. In particular, robot-aided rehabilitation proved to be an effective tool for providing high-intensity treatments to patients suffering from neurological and musculoskeletal disorders.

Despite the fact that continuous-passive motion machines can effectively improve the range of motion of specific anatomical districts, more complex robotic platforms may play a paramount role in increasing patient active participation during therapy since they can induce neural plasticity to speed up motor recovery. Indeed, robotic architecture can include different features that aim at engaging the patient in interacting with the machine. Assist-as-needed controllers are designed to provide minimal assistive forces to the patient in such a way that the robot should intervene only if the patient is not capable of performing the task autonomously. Furthermore, the inclusion of the patient's intention in the control loop, i.e. to trigger the initiation of movement, results in successful clinical outcomes. Lastly, the gamification of the rehabilitation process thanks to the virtual reality technologies allows engaging the patients while they are doing motor exercises.

Although all these works stress the importance of involving and engaging patients undergoing robot-aided treatments, a methodology to continually estimate to what extent the patient is actively participating in performing the motor exercise and leveraging such metrics inside the robot control loop has not been addressed in the scientific literature.

The works in J. Wagner, T. Solis-Escalante, P. Grieshofer, C. Neuper, G. Muller-Putz, and R. Scherer, "Level of participation in robotic-assisted treadmill walking modulates midline sensorimotor eeg rhythms in able-bodied subjects," Neuroimage, vol. 63, no. 3, pp. 1203-1211, 2012 and G. Tacchino, M. Gandolla, S. Coelli, R. Barbieri, A. Pedrocchi, and A. M. Bianchi, "Eeg analysis during active and assisted repetitive movements: evidence for differences in neural engagement," IEEE Transactions on Neural Systems and Rehabilitation Engineering, vol. 25, no. 6, pp. 761-771, 2016 evidenced that some distinctive features of the electroencephalogram (EEG) significantly change between active and passive walking with a lower-limb exoskeleton and in performing upper limb repetitive motions. The experiment carried out in E. Koyas, E. Hocaoglu, V. Patoglu, and M. Cetin, "Detection of intention level in response to task difficulty from eeg signals," in 2013 IEEE International Workshop on Machine Learning for Signal Processing (MLSP). IEEE, 2013, pp. 1-6 found that EEG signals can be used to extract the intention level of the subjects in response to task difficulty. Moreover, the authors evidenced that some correlations between cortical and muscular activity exist when the participants exert different levels of participation. However, the instrumentation used in these works is wearable but requires extensive calibration procedures and can be considered invasive, and wearing an EEG helmet is not feasible for daily rehabilitation therapy.

Wearable monitoring seems to be a valuable alternative to monitoring user physiological signals. Muscular activity is measured in S. Pareek, H. Manjunath, E. T. Esfahani, and T. Kesavadas, "Myotrack: Realtime estimation of subject participation in robotic rehabilitation using semg and imu," IEEE Access, vol. 7, pp. 76 030-76 041, 2019. Healthy subjects were enrolled to undergo an experiment to develop a binary classifier to predict whether a participant is passively or actively tracking a displayed trajectory with a haptic device. Wearable surface electromyography (sEMG) resulted to be an optimal estimator of patient participation. On the other hand, only sEMG is taken into account in the developed model. Moreover, the present invention may allow the prediction of only the discrete class between active and passive movement.

Some efforts have been made on robot control to close the loop on the patients themselves in the so-called biocooperative systems. Metrics can be computed during the rehabilitation session in order to tune the parameters of the controller in real-time. For instance, kinematics performance as well as patient physiological parameters can be used in gait, and in upper limb robot-aided rehabilitation. On the other hand, all these controllers aim at involving more and more participants without an explicit quantification of patient participation.

To overcome this limitation, the present invention aims at proposing a novel technique to quantify patient active level of participation (ALP) during upper-limb robot-aided rehabilitation and a control strategy to take into account the estimated ALP. An unobtrusive multimodal interface was developed and integrated into an end-effector rehabilitation robot to monitor sEMG and pressure exchanged between the robot and the user. The computation of the ALP enables the development of increasingly human-centered robotic platforms capable of real-time decision-making through patient multimodal monitoring. The proposed ALP-adapting robot was compared with respect to a state-of-the-art impedance controller to assess the closing of the control loop on such human-centred metrics.

Below, firstly, details are provided of an exemplary embodiment of the present invention, and experiments for the validation thereof is described. Then, results are presented that were obtained during said experiment. Lastly, a main conclusion for these experiments is made.

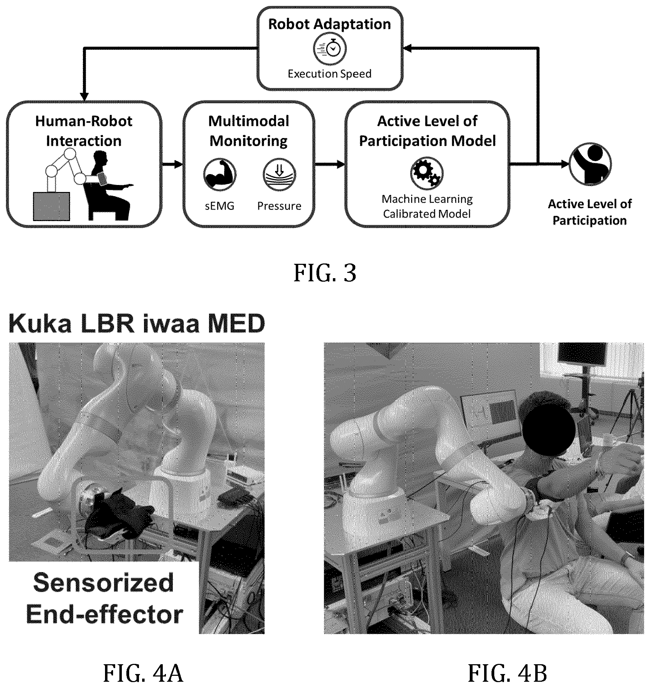

FIG. 3 presents the block scheme of a proposed approach as used in the present example and which is in accordance with embodiments of the present invention. During a session of upper-limb robot-aided rehabilitation, a multimodal monitoring system can be designed in order to measure biomechanical and physiological information of the human-robot interaction. In particular, the sEMG is one of the most commonly used measures to assess user motion intention. On the other hand, the human-robot interface can be exploited to sense the pressure the user exerts on the robot end-effector.

These quantities are capable of providing a picture of the human-robot interaction to quantify to what extent the user is participating in accomplishing the motor task. Machine Learning algorithms can be trained to identify the two extreme conditions, e.g., the patient not participating at all and the active participation. In order to return a continuous estimate of the active level of participation (ALP), model calibration is needed. Thus, an experiment is needed to collect data to capture the behavior of healthy participants to model the ALP. The computed ALP can be used to adapt the robot behavior.

In particular, the proposed approach adapts the task execution speed according to the ALP. The human-robot interaction designed in this example along with the developed multimodal monitoring interface and the active participation model, presented in FIG. 3, are detailed in the following.

The interaction between the robot and the user is a conventional Cartesian impedance controller around a set point. The robot motion dynamics along with the implemented control law are given by:

$$\tau_C = \mathbf{B}(\mathbf{q})\mathbf{y} + \mathbf{C}(\mathbf{q}, \mathbf{q}^{\cdot})\mathbf{q}^{\cdot} + \mathbf{F}_V \mathbf{q}^{\cdot} + \mathbf{F}_s sign(\mathbf{q}^{\cdot}) + \mathbf{g}(\mathbf{q})$$

$$\mathbf{y} = \mathbf{J}^{\dagger}(\mathbf{q}) \cdot \mathbf{K} \cdot \tilde{\mathbf{x}}$$

where $\mathbf{B}(\mathbf{q})$ is the robot inertia matrix, $\mathbf{C}(\mathbf{q}, \mathbf{q})$ accounts for Centrifugal and Coriolis effects, $\mathbf{F}_V$ is the viscous friction torque, $\mathbf{F}_s sign(\mathbf{q})$ is the static friction torque, $\mathbf{g}(\mathbf{q})$ is the gravity contribution, $\mathbf{q}$, $\mathbf{q}'$ and $\mathbf{q}$ are the robot joint position, angular velocity and acceleration, respectively, $\tau_C$ is the torque supplied by the actuators and $\mathbf{y}$ is the control law. In particular, $\mathbf{J}^{\dagger} = \mathbf{J}^T \mathbf{J} \cdot \mathbf{J}^T {}^{-1}$ is the right pseudo-inverse of the robot geometric Jacobian, $\mathbf{K}$ is the stiffness matrix and $\tilde{x}$ = $x_d$ - $x_a$ represents the pose error between the desired pose $x_d$ and the current pose $x_a$. The desired speed and acceleration ($\mathbf{x}_d$ and $\mathbf{x}_d$) are not considered inside the control law presented in the above equations since the controller of this example is adapted to provide a set point without an explicit time law.

In order to acquire the specific trajectory to be replayed by the robot in a specific session, the robot can be set transparent by defining the current stiffness matrix as $\mathbf{K}$ = $diag\{0, 0, 0, 0, 0, 0\}$ N/m. Once the recording starts, the users can freely move their arm attached to the robot end-effector and the Cartesian position and orientation are saved in the reference demonstration $x_{demo}$. The set-point $x_d$ introduced in the control law in the above equations will be $x_d \in x_{demo}$.

The human-robot interface developed for this example is composed of two sensing modalities: pressure and EMG. The development of the interface is described in detail in K. Langlois, E. Roels, G. Van De Velde, C. Espadinha, C. Van Vlerken, T. Verstraten, B. Vanderborght, and D. Lefeber, "Integration of 3d printed flexible pressure sensors into physical interfaces for wearable robots," Sensors, vol. 21, no. 6, p. 2157, 2021 and K. Langlois, J. Geeroms, G. Van De Velde, C. Rodriguez-Guerrero, T. Verstraten, B. Vanderborght, and D. Lefeber, "Improved motion classification with an integrated multimodal exoskeleton interface," Frontiers in Neurorobotics, p. 140, 2021. The EMG electrodes are made of conductive textile (EeonTex NW170-PI-20).

As already indicated above, assessing the ALP of patients during robot-aided rehabilitation is preferred in order to engage them more and more in exercising with the robot. ALP is a measure that encompasses different areas related to both the physical and cognitive spheres. In particular, we may define ALP as a combination of i) physical workload, ii) intention, iii) performance and iv) engagement.

From a physical point of view, if the patients are actively performing the rehabilitation task, they exert a certain physical workload. On the other hand, if the patient slacks, the robot has to assume the leading role in accomplishing the task since the patient is not providing any contribution. The interaction forces can be useful to assess the amount of workload exchanged between the user and the machine. Moreover, another ALP feature is the intention to move. The participation of patients in exercising themselves represents also the will of performing the task with respect to being guided along it. The patient intention relates to the EMG.

Moving towards the cognitive sphere, participation appears to be closely linked with performance. A subject who wants to perform well actively performs the assigned exercise. The performance during rehabilitation is related to the accuracy of tracking the desired motion. At last, engagement in what one is doing and the perception of the interaction also play a paramount role in ALP.

To this purpose, the ALP can be predicted starting from the measures coming from the multimodal interface. In particular, a structured data acquisition campaign is typically needed to train a machine learning model. Given a temporal window of 1 second, statistical features such as the mean, standard deviation, minimum, maximum, and mean value of the first and second derivative can be computed from the four channels of the measured pressures. From the raw EMG signal, both time and frequency domain features were computed as described in K. Langlois, J. Geeroms, G. Van De Velde, C. Rodriguez-Guerrero, T. Verstraten, B. Vanderborght, and D. Lefeber, "Improved motion classification with an integrated multimodal exoskeleton interface," Frontiers in Neurorobotics, p. 140, 2021 and F. Leone, C. Gentile, F. Cordella, E. Gruppioni, E. Guglielmelli, and L. Zollo, "A parallel classification strategy to simultaneous control elbow, wrist, and hand movements," Journal of NeuroEngineering and Rehabilitation, vol. 19, no. 1, pp. 1-17, 2022. In particular, the root mean square, the average amplitude change, variance, integrated EMG, average energy, wavelength, mean absolute deviation, and logarithmic difference of absolute mean values are extracted as time-domain features. Moreover, mean and median frequencies are taken into account. To sum up, 10 and 24 features are extracted from the EMG and pressures, respectively, for a total of 34 features. Once the dataset has been collected, machine learning models can be fed with the data to classify the two different labels, e.g. passive and active participation. Since, in this example, it is tried to provide a framework to estimate in a continuous manner the ALP and not simply the binary class participating or not, a machine learning model calibration step is preferred (see B. Zadrozny and C. Elkan, "Transforming classifier scores into accurate multiclass probability estimates," in Proceedings of the eighth ACM SIGKDD international conference on Knowledge discovery and data mining, 2002, pp. 694-699). Calibration is an essential step for a machine learning model used in a clinical setting since it can provide the probability that its advice is right, e.g., to what extent the participant is actively participating or not. In particular, the isotonic regression calibration approach was used in the present example, as described in A. Niculescu-Mizil and R. Caruana, "Predicting good probabilities with supervised learning," in Proceedings of the 22nd international conference on Machine learning, 2005, pp. 625-632. It maps the posterior probability of a supervised learning model to a monotonically increasing function. Given the predictions of the model $f_i$ and the true targets $y_i$, isotonic regression assumes that

$$y_i = m(f_i) + \epsilon_i$$

where m(.) is a monotonically increasing function and $\varepsilon_i$ represents a residual. A minimization problem can be carried out in order to find $m(\cdot)$ defined as

$$\hat{m} = argminz \sum_i (yi - z(fi))$$

where $(f_i, y_i)$ is the $i$-th sample of a calibration dataset.

Once an estimation of the ALP is available, it is possible to take it into account to adapt the robot behavior. The present exemplary approach aims at modifying the task execution speed according to the ALP of the participants. Given the recorded trajectory $x_{demo}$, composed of T samples, at each iteration, the ALP is used to compute the number of samples $\Delta t$ to skip in the recorded trajectory $x_{demo}$ in order to assign a reference set-point to the robot as

$$x_d(t + 1) = x_{demo}(t + \Delta t)$$

where $\Delta t$ is defined as

$\Delta t = 0$ if ALP < $ALP_{cal}$
$\Delta t = 2$ if $ALP_{cal} \le$ ALP < mean ($[ALP_{cal,}\ 1]$)
$\Delta t = 4$ if ALP $\ge$ mean ($[ALPcal, 1]$)

where $ALP_{cal}$ is a subject-specific threshold computed in a calibration phase. In this way, the lower the ALP, the lower the $\Delta t$, and the higher the time needed to complete the replay of the demonstrated trajectory. If the participant slacks, e.g. the ALP is $\le ALP_{cal}$, the robot will not move until the model estimate a user ALP greater than $ALP_{cal}$.

In order to validate the proposed approach, an experiment was designed and carried out by enrolling 15 healthy participants (10 males and 5 females, 34.5 $\pm$ 14.18 mean age). All of them signed a written consent to participate in this experiment. In particular, two experimental sessions were carried out. The first one aimed at recording data about the human-robot interaction to train the ALP machine-learning estimation model. Once the model is validated offline, the second experiment was carried out in order to assess the ALP-adapting robot impact on the participants with respect to a simple one.

FIG. 4A and 4B present the experimental setup used in this study. The Kuka iiwa LBR Med, reported in FIG. 4A, is the robotic device used to provide motor therapy. The motion the robot executes during the experiment is recorded using Programming by Demonstration. The subject is strapped into the human-robot interface, and the motion is recorded by moving the patient arm, with the robot attached. Using a pedal, an indication is given that a demonstration has started, and at the release has ended. During demonstration, Cartesian poses (i.e. position and orientation) of the end-effector are recorded and are replayed during the experiment.

In the first experiment, five participants are asked to perform shoulder flexion/extension (sFE) exercise with the robot aid.

The volunteers are asked to sit comfortably near the rehabilitation robotic platform. The participants are asked to slip their right arm into the human-robot interface to fit in it. As explained above, when the participants are comfortably attached to the robot end-effector, they record sFE movement with the robot transparent to collect $x_{demo}$. When the demonstration is recorded, the replay of the current motion takes place. In both the experimental conditions, the stiffness matrix of the robot is set at $\mathbf{K} = diag\{500, 500, 500, 300, 300, 300\}$ N/m. These values are in fact typically used in end-effector robotic platforms for upper limb rehabilitation. The participants are asked to perform 30 repetitions of the recorded motion in two experimental conditions:

- Passive Participation (PP): the users are slacking and let the robot guide their arm in accomplishing the motor task. They passively interact with the robot.
- Active Participation (AP): the volunteers actively attempt to follow the trajectory. They participate in following the reference path.

Linear Discriminant Analysis (LDA), linear Support Vector Machine (SVM), Logistic Regression (LR), and k-Nearest Neighbours (kNN), with $k = 1$, classifiers were compared.

In the second experimental validation phase, the ALP estimation module is used in real-time to adapt the robot behavior. To prove the effectiveness of the proposed ALP-adapting robot, two groups of five participants each were enrolled. In particular, all the participants were asked to perform 30 repetitions of the sFE task without providing further information to avoid bias in the groups. At the beginning of the experiment, 15 repetitions are performed with fixed $\Delta t = 4$ samples in order to measure the participant baseline. In this phase, the aforementioned subject-specific threshold $ALP_{cal}$ is computed as the mean of the ALP collected during the first 15 repetitions of the rehabilitation treatment.

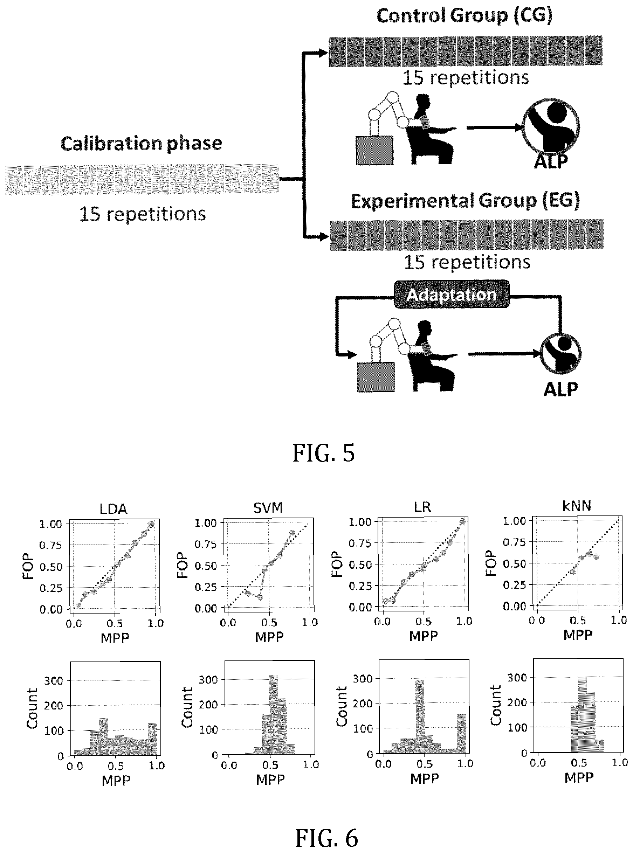

The rest of the experiments were performed following a double-blinded strategy: neither the participants nor the researcher knows which controller is provided to the participants. The control group (CG) interacts with the not adapting robot, e.g. fixing $\Delta t = 4$ samples. The experimental group (EG) undergoes the rehabilitation session with the ALP-adapting robot. A schematic representation of the second experimental session is provided in FIG. 5.

The first experimental phase assesses the machine learning models capability in estimating the ALP. K-fold cross- validation ($k = 5$) is carried out on the collected dataset to compute the performance of the implemented classifiers in accurately predicting both the label and the score. In particular, the training set per each folder is composed of the 80% of the dataset and the i-th fold (20%) is split up into a validation set (10%), used to calibrate the classifiers, and a test set (10%), on which the following metrics are computed:

- Accuracy: defined as the proportion of the correct predictions with respect to the total number of tested samples. The higher the accuracy, the higher the model performance.
- Brier Score (BS): it measures the accuracy of probabilistic predictions as

where $N$ is the number of testes samples, $f_i$ is the predicted value and $o_i$ is the observed one. The BS computes the mean squared error between the predicted probabilities and the observed values. The lower the BS, the better the predictions are calibrated.

The best performing model among the tested ones, e.g. LDA, SVM, LR, and kNN, will be run in the second experimental phase as the ALP model in real-time. In the second experimental phase, the ALP-adapting robot is compared with respect to the not-adapting condition by means of a set of performance indicators assessing the biomechanics of the human-robot interaction as well as the subjective perception of the controllers.

- ALP: since the proposed controller is taking into account the ALP, the estimated ALP value can be used as a performance indicator itself to assess the impact of the proposed ALP adapting robot on the user.
- Robot work ($W_R$): the work done by the robot to perform a single movement is used as an indicator of the mechanical work that the user has put into the system. It is computed as where $T$ is the number of samples collected in the experiment, and $F$ and $v$ are the total interaction force sensed by the robot and the end-effector speed in Cartesian space computed at the time instant $t$, respectively. The dot product of these two quantities allows computing the current power of the system. The work is retrieved by integrating it over time. A positive work means that the interaction force $F$ is applied in the same direction as the performed motion $v$. The higher the $W_R$ the higher the energy that the participant put inside the system. A slacking participant would apply a force that opposes the movement imposed by the robot and consequently, $W_R$ will be negative. The $W_R$ is computed only during the shoulder flexion, e.g. the rising phase of the task, since in this phase the participants had to actively compensate the gravitation force to raise their arm.
- Trajectory Error (TE): the error in following the reference position is computed as where $T$ is the number of samples collected in a session phase and $p_d$ and $p_a$ represent the desired and current position in Cartesian space at the $t$-th time stamp. The higher the user performance, the lower the *TE.*

- Integrated EMG (iEMG): since the proposed ALP-adapting robot wants to stimulate the participant to play the main role, the movement intention of the participant was measured by extracting the integral of EMG signal. In particular, the EMG was preprocessed by means of a bandpass 4$^{th}$ order Butterworth filter in the range [15 - 400] Hz. Moreover, the signal was rectified and a zero-lag 100 ms moving average filter was applied to compute the enveloped EMG (EnEMG). The iEMG is computed as where EnEMG is the enveloped EMG, T represents the total number of samples collected in an experimental phase, and t indexes the t-th sample. The higher the iEMG, the higher the intention exhibited by the par- ticipant in interacting with a certain controller.

In order to highlight the impact of the robot adaptation on the participants, the aforementioned performance indicators computed during the second 15 repetitions were normalized with respect to the values observed in the calibration phase as where X is one among {ALP, WR, TE, iEMG} and Xcal is the mean value of the metrics computed in the calibration phase. Moreover, the subjective perception of the controllers on the participants is assessed by means of questionnaires. The engagement in interacting with the robot was assessed by means of the Self Assessment Mannequin (SAM) questionnaire. The SAM allows the participants to declare their Valence of the response (from positive to negative), perceived Arousal (from high to low levels), and perceived Dominance (degree of control that a person has over the situation) evoked by rehabilitation robot use (see T.-M. Bynion and M. T. Feldner, Self-Assessment Manikin. Cham: Springer International Publishing, 2017, pp. 1-3. [Online]. Available: https://doi.org/10.1007/978-3-319-28099-8 77-1). Moreover, the NASA-TLX was administered to assess the perceived workload in interacting with the robot in the two experimental conditions (see L. M. A. La Bara, L. Meloni, D. Giusino, and L. Pietrantoni, "Assessment methods of usability and cognitive workload of rehabilitative exoskeletons: A systematic review," Applied Sciences, vol. 11, no. 15, p. 7146, 2021). In particular, the participants were asked to rate from 0-10 their experience in terms of Mental Demand (MD), Physical Demand (PD), Temporal Demand (TD), Performance (PER), Effort (EF), and Frustration (FR).

To assess the impact of the user ALP-adapting robot use with respect to the simple impedance controller, a statistical analysis has been carried out on the collected data. In particular, the Wilcoxon rank-sum test is performed on the aforementioned performance indicators for the two groups of participants: CG and EG henceforth. This test assesses whether a significant difference exists between the two investigated conditions. The significance level is set at p-value $\leq 0.05$.

FIG. 6 reports the results of the first experimental phase. In particular, the calibration plot, shown in the first row, presents the mean predicted probabilities (MPP) vs the fraction of positives (FOP) returned by the four tested machine learning approaches. The bisector line represents a perfectly calibrated model. Moreover, the second row reports the histogram of the occurrences of the MPP. The calibration procedure aims at improving the probability score estimation returned by the machine learning classifiers. As evident from the calibration plots, SVM and kNN are not able to cover the entire range [0,1] of predicted probability. This means that is very unlikely that these two classifiers return a correct ALP score ranging from the complete passive condition to the complete active one. On the other hand, LDA and LR are capable of covering all the MPP range. Furthermore, the histogram of the MPP occurrences provides additional information. A well-calibrated model must not be unbalanced in predicting the same MPP every time. SVM, LR, and kNN calibrated classifiers exhibited a normal distribution like MPP. On the contrary, LDA is not biased to return the same value, and thus it resulted to be the closest to a uniform distribution. Moreover, the performance of the tested models is reported in Table I.

Table I: Calibrated models performance; results of the first experimental phase.

|  | Accuracy [%] | Brier Score |
|---|---|---|
| LDA | 75.30 $\pm$ 6.88 | 0.15 $\pm$ 0.01 |
| SVM | 59.81 $\pm$ 4.11 | 0.23 $\pm$ 0.01 |
| LR | 70.66 $\pm$ 5.75 | 0.17 $\pm$ 0.01 |
| kNN | 58.52 $\pm$ 2.62 | 0.24 $\pm$ 0.01 |

It is worth highlighting that the LDA outperformed the other approaches since returned the highest accuracy and the lowest Brier score. Consequently, LDA was selected as the ALP estimation model in the second experimental phase.

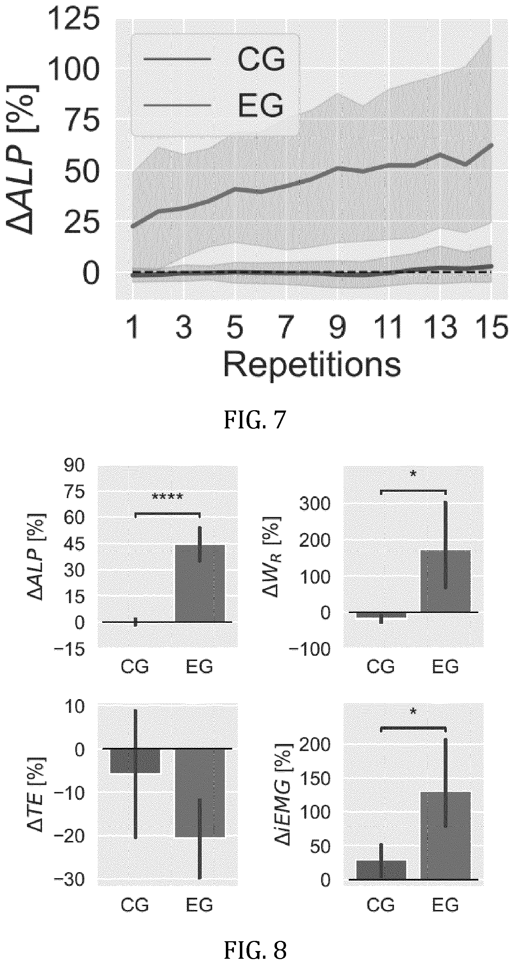

The $\Delta ALP$ estimated during the last 15 repetitions of the experiment for the two groups is reported in FIG. 7 over time. In particular, the solid lines represent the mean value per each group and the shaded areas stand for the 95% confidence intervals. It is worth evidencing that the CG did not exhibit any modification of the ALP over time since the robot behavior did not change. The participants who interacted with the ALP- adapting robot exhibited an increasing trend of the ALP over the repetitions. That means that the participants understood that the robot reacts to their ALP and tried to increase their participation over time to let the robot move and complete the assigned task, e.g. executing 30 repetitions of the sFE.

The $\Delta ALP$ along with the other performance indicators, introduced above, are reported in FIG. 8. The bar plots show the mean value of the indicator computed for the two participant groups and the solid black line stands for the 95% confidence intervals. Moreover, the statistically significant differences are highlighted by means of asterisks:

{*} for $0.01 \leq$ p-value $\leq 0.05$,
{**} for $1 \cdot 10^{-3} \leq$ p-value $\leq 0.01$, and
{* * **} for p-value $\leq 1 \cdot 10^{-3}$.

As already shown in FIG. 7, the $\Delta ALP$ significantly increased in the second part of the experiment only for the participants who interacted with the ALP-adapting robot (EG). The physical workload exerted by the participants is computed in the flexion phase of the sFE exercise by computing the $W_R$ to assess to what extent the participants are capable of compensating the gravitational force to rise their arm. The $\Delta W_R$ significantly increased during the second part of the session, it assumed -32.91 $\pm$ 29.17 J during the calibration to reach 4.35 $\pm$ 39.15 J in the experimental phase. That means that the EG participants started to put more energy into the system. On the other hand, the CG exhibited a slight decrease in the $\Delta W_R$, e.g. -43.47 $\pm$ 32.29 J and -53.00 $\pm$ 42.83 J in the calibration and experimental phases, respectively. In other words, the CG participants slaked their arms more and more in interacting with the robot since the interaction force, during the shoulder flexion, is pointing in the opposite direction to the velocity. Conversely, EG participants started to push in the same direction of the movement in order to let the robot move. The performance of the two experimental groups, in terms of TE, was comparable. The errors in tracking the demonstrated path were $(7.3 \pm 6.8) \cdot 10^{-3}$ m and $(5.4 \pm 3.7) \cdot 10^{-3}$ m for the CG and EG respectively. The participants interacting with the ALP-adapting robot exhibited a non-statistically significant improvement in their performance as soon as the robot started reacting to them. In particular, during the flexion of the participants shoulders, they were capable of reaching higher positions on their own.

Muscle activity was also affected by the robot adaptation. Indeed, the iEMG metrics significantly increased in the experimental condition of EG participants with respect to the calibration phase. The overall increased muscular activation is a key factor for assessing the user intention in participating in the rehabilitation training. Despite the CG shown a slight increase with respect to the calibration phase, the $\Delta iEMG$ of the EG increased by about 130% (p-value= 0.01).

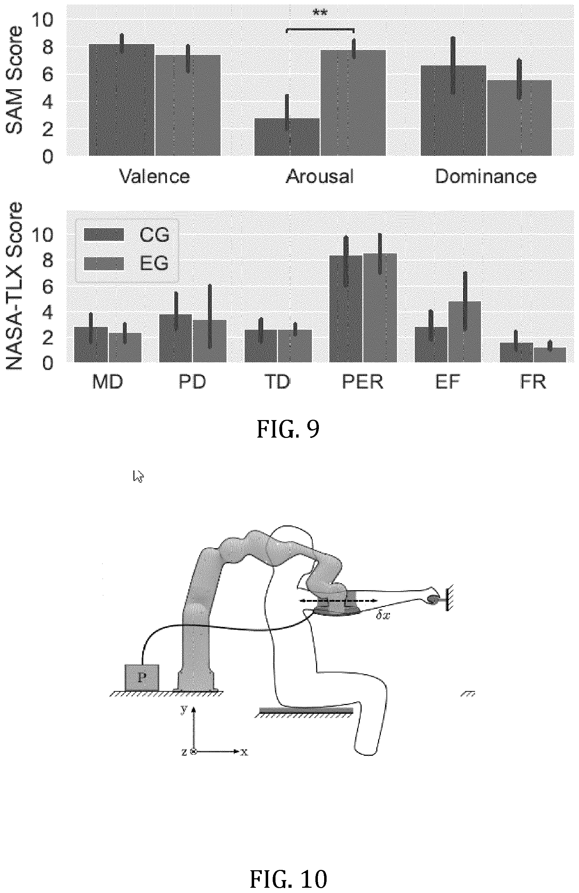

Finally, the results on the level of engagement and perceived workload are shown in FIG. 9. From the SAM questionnaire, it is evident that both the experimental groups experienced positive feelings and were confident in interacting with the robot, e.g., high values of Valence and Dominance, respectively. On the other hand, the Arousal ranking of the two groups was significantly different (p-value= 0.007). That means that interacting with a rehabilitation robot that adapts according to the participant ALP significantly improves the perceived excitement. The NASA-TLX questionnaire revealed that the two robotic platforms are comparable from a perceived workload point of view. It is noteworthy the difference in the effort perception even if it is not statistically significant (p-value= 0.31). EF was rated 2.8 $\pm$ 1.5 and 4.8 $\pm$ 3.0 by CG and EG, respectively. The ALP-adapting robot is more challenging than the non-adapting one since it requires the participants more effort to proceed with the therapy.

The present example has provided a methodology in accordance with embodiments of the present invention to objectively estimate the ALP of participants interacting with a robot during robot-aided rehabilitation sessions and test it inside the control loop of an ALP-adapting robot. The estimation model is fed by a multimodal monitoring interface that senses the electromyographic activity of the biceps and the pressure exchanged at the human-robot interface. It is worth noticing that the proposed method is validated onto a specific robotic platform and movement of the upper limb, i.e., the shoulder flexion/extension, but the procedure described in this paper can be extended to different robots and motions. Indeed, the ALP estimation method relies only on the measurements collected at the human-robot interface, where the interaction itself takes place.

At first, a machine-learning model calibration procedure was performed in order to improve the probability prediction of commonly used classifier starting from data acquired from five healthy participants. LDA resulted to be the most suitable in terms of classification accuracy and goodness of calibration, e.g. the lowest Brier score.

The trained model was used then to estimate in real-time the ALP of two groups of five participants each interacting with a rehabilitation robot, e.g. namely the CG and EG. The participants who interacted with the ALP-adapting robot exhibited a significantly higher physical workload, muscular activity, and level of perceived excitement with respect to the CG. This demonstrates that closing the robot control loop on the participants effectively enhances the interaction stimulating the users to provide more and more effort in exercising themselves.

The proposed approach could be applied during robot-aided rehabilitation sessions of pathological subjects to assess the capability of the ALP estimation model to identify the ALP of patients with limited motor functions. Moreover, the ALP model could exploit different sensing modalities to take into account measures that relate to the cognitive workload of the users.

In a second example, the possibilities of embodiments of the present invention are shown with respect to the detection of human intention in wearable robots, such as for example exoskeletons. Exoskeleton controllers often assume full transmission of actuators' torque to the human body, neglecting torque loss. According to some embodiments of the present invention, 3D force sensing is applied. It thereby is an advantage that the analysis of arm movements is less restricted, compared to measuring only normal force, using a 1D force sensor. 3D force sensing can be very sensitive and can be integrated in the physical interaction device according to embodiments of the present invention, to verify, in real-time, the efficiency of force and torque transmissions. The additional advantages of assessing shear forces are discussed, as well as a classification algorithm for touch and motion. In the present example a setup is chosen wherein twelve 3D force sensors are used. These are integrated in a cuff, forming the physical interface device between a robot and the user. The 3D force sensors used may be any suitable 3D force sensors. The translation and rotation frame of the 3D force sensors is thereby referenced to the center of mass frame of the physical interface device. The sensors in the present example are arranged as three parallel lines of four 3D sensors (each set of four 3D sensors thus being aligned on a single line), although other arrangements also may be used.

Controlling of the robot movement could be performed in two ways, using the data obtained with the 3D force sensing. One way is referred to a using admittance control, whereby the velocity of the end-effector is controlled. The mathematical model behind this can be expressed by the following equation :

$$\begin{bmatrix} F_x \\ F_y \\ F_z \\ T_x \\ T_y \\ T_z \end{bmatrix} = \begin{bmatrix} m_{1x} & m_{1y} & m_{1z} & I_{1x} & I_{1y} & I_{1z} \\ m_{2x} & m_{2y} & m_{2z} & I_{2x} & I_{2y} & I_{2z} \\ m_{3x} & m_{3y} & m_{3z} & I_{3x} & I_{3y} & I_{3z} \\ m_{4x} & m_{4y} & m_{4z} & I_{4x} & I_{4y} & I_{4z} \\ m_{5x} & m_{5y} & m_{5z} & I_{5x} & I_{5y} & I_{5z} \\ m_{6x} & m_{6y} & m_{6z} & I_{6x} & I_{6y} & I_{6z} \end{bmatrix} \begin{bmatrix} \dot{V}_x \\ \dot{V}_y \\ \dot{V}_z \\ \dot{\omega}_x \\ \dot{\omega}_y \\ \dot{\omega}_z \end{bmatrix} + \begin{bmatrix} c_{1x} & c_{1y} & c_{1z} & c_{r1x} & c_{r1y} & c_{r1z} \\ c_{2x} & c_{2y} & c_{2z} & c_{r2x} & c_{r2y} & c_{r2z} \\ c_{3x} & c_{3y} & c_{3z} & c_{r3x} & c_{r3y} & c_{r3z} \\ c_{4x} & c_{4y} & c_{4z} & c_{r4x} & c_{r4y} & c_{r4z} \\ c_{5x} & c_{5y} & c_{5z} & c_{r5x} & c_{r5y} & c_{r5z} \\ c_{6x} & c_{6y} & c_{6z} & c_{r6x} & c_{r6y} & c_{r6z} \end{bmatrix} \begin{bmatrix} V_x \\ V_y \\ V_z \\ \omega_x \\ \omega_y \\ \omega_z \end{bmatrix}$$

in combination with a virtual spring. The mathematical meaning of this equation is known to the person skilled in the art, but can for example also be found in Sharkawy et al. "Human-Robot Interaction : A review and Analysis on Variable Admittance Control, Safety, and Perspectives (2022) Machines 10(7):591, 1 - 26. The admittance control takes as input the forces obtained from the force sensors and as output the movement of the end effectors. Using data based control, like for example using a neural network or fuzzy logic, allows for variable admittance control.

A second way of controlling is referred to as impedance control, whereby the torque of the joints is controlled. The latter is applicable in case of small velocity and acceleration. The mathematical model behind this can be expressed as

$$\tau = \tilde{g}(\theta) + J^T(\theta)\left(\mathcal{F}_d + K_{fp}\mathcal{F}_e + K_{fi}\int \mathcal{F}_e(t)dt\right)$$

The mathematical meaning of this equation is known to the person skilled in the art, but can for example also be found in Lynch et al., Video Supplements for Modern Robotics, Cambridge University Press (chapter 11.5, force control), hyperlink https://modern robotics.northwestern.edu/nu-gm-book-resource/11-5-force-control/#department. The impedance control takes the movement of the arm and variation in the force sensor value as input and, using a PI controller, provides the torque joint value required to come back to the initial force value, thus allowing to control. So starting from a reference value of the force sensor, the movement to come back to the reference value is determined with a PID controller and used for controlling the robot. The latter allows for good control, whereby stiffness and overdamping can be taken into account. It allows further to differentiate between internal and external force.

Since the sensing according to embodiments of the present invention was performed in direct contact with the limb, there is no need for taking into account the environment, so that affection of the results by environmental noise can be avoided.

In the example, neural network classification mechanisms were used to estimate the effect of the importance of the shear component and to identify the relevance of the sensor positions used. Furthermore, neural network algorithms were also used to perform classification of the motions performed in real time. It was found that accurate classification was possible.

Furthermore, shear force was also used to detect relative motion between the arm and the physical interface device. Such motion is illustrated in FIG. 10. Shear force not only could be used to identify relative motion, but moreover to also quantify it. The principle of using shear force was explained in Langlois K., Rodrigues Cianca D., errien, B., De Winter, J., Verstraten, T., Rodriguez Guerrero, C. D., Vanderborght, B., & Lefeber, D., in "Investigating the effects of strapping pressure on human-robot interface dynamics using a soft robotic cuff", IEEE transactions on medical robotics and bionics. (2020), 3(1), 146-155.

It is to be understood that although preferred embodiments, specific constructions and configurations, as well as materials, have been discussed herein for devices according to the present invention, various changes or modifications in form and detail may be made without departing from the scope of this invention. For example, any formulas given above are merely representative of procedures that may be used. Functionality may be added or deleted from the block diagrams and operations may be interchanged among functional blocks.

**Reference numbers**

| | |
|---|---|
| 101 | integrated pressure sensors |
| 102 | integrated textile EMG electrodes |
| 103 | magnetic snap buckles |
| 104 | boa strapping |
| 200 | machine, e.g. cobot |
| 201 | physical interface device (e.g. sensorized cuff) |
| 202 | trolley |
| 203 | pedal |
| 204 | patient safety button |
| 208 | soft material |
| 210 | one or more integrated sensors, at least one being configured for sensing a force inside the physical interface device |
| 212 | data output |

**Claims**

1. A physical interface device (201) for providing a physical interface between a user and rigid components of a machine (200), the physical interface device (201) comprising

   - a soft component (208) for providing a soft contact to the user, and
   - one or more sensors (210) which are integrated in the physical interface device (201), wherein at least one of the sensors (210) is configured for sensing a force inside the physical interface device (201).

2. A physical interface device (201) according to claim 1, wherein the device (201) comprises an output (212) or is configured to provide data to an output (212) for indicating information regarding the application of the physical interface device (201) to the user or regarding movement or movement intentions by the user, based on data of the one or more sensors (210) integrated in the physical interface device (201).

3. A physical interface device (201) according to any of claims 1 or 2, wherein one or more sensors (210) are configured so as to provide the ability to measure biological signals directly on the human skin, and/or wherein the one or more sensors (210) comprise one or more force and/or pressure sensors.

4. A physical interface device (201) according to claim 3, wherein the one or more force and/or pressure sensors are configured for measuring a pressure distribution on skin tissues.

5. A physical interface device (201) according to any of claims 3 or 4, wherein the one or more sensors are configured for 3 dimensional tactile force sensing.

6. A physical interface device (201) according to any of the previous claims, wherein the one or more sensors (210) comprise one or more sensors for obtaining a force in three directions, inside the physical interface device (201), and/or

   wherein the one or more sensors (210) comprise a plurality of force sensors for obtaining a force in three directions at a plurality of positions inside the physical interface device, and/or
wherein the one or more sensors (210) comprise one or more sensors for capturing the intention of the user for performing a certain movement.

7. A physical interface device (201) according to any of the previous claims, wherein the one or more sensors (210) comprise one or more electromyography (EMG) sensors, and/or wherein the one or more sensors (210) combine at least one electromyography sensor, at least one pressure sensor and at least one inertial measurement sensor.

8. A physical interface device (201) according to any of the previous claims, the physical interface device (201) comprising releasable connections for releasebly connecting the physical interface device to components, e.g. a robotic arm, of the machine.

9. A machine (200) comprising a physical interface device (201) according to any of the previous claims.

10. A machine (200) according to claim 9, the rehabilitation robot comprising one or more force sensors for sensing force (e.g. force components in one, two or three directions) which is applied externally to the physical interface device.

11. A machine (200) according to any of claims 9 or 10, the machine (200) being

- a rehabilitation robot, or
- a rehabilitation robot being an end-effector robot, or
- a rehabilitation robot for upper-body extremities and/or lower-body extremities, or
- a rehabilitation robot using a robotic arm as actuation device, or
- a rehabilitation robot using a robotic arm as actuation device having 6 spatial degrees of freedom and being able to alter over time.

12. A machine (200) according to any of claims 9 or 10, the machine being an exoskeleton.

13. A physical interface device (201) according to any of claims 1 to 8 or a machine (200) according to any of claims 9 to 12, the device or machine comprising or being configured to communicate with a user interface, for giving feedback to the user, or
wherein the device (201) or machine (200) is adapted for performing data analytics, including providing data or processing data, the data comprising one or more of pressure readings, EMG readings, machine peak force, machine current position and programmed reference trajectory, amount of transferred work between patient and robot, level of active participation, cuff strapping pressure and performance of patient.

14. A physical interface device (201) or a machine (200) according to claim 13, wherein the device (201) or machine (200) is adapted for combining internal force in the physical interface device with external force on the physical interface device (201).

15. A physical interface device (201) or machine (200) according to claim 14, wherein the combined internal force and external force is used for determining the data comprising one or more of pressure readings, EMG readings, robot peak force, robot current position and programmed reference trajectory, amount of transferred work between patient and robot, level of active participation, cuff strapping pressure and performance of patient.

FIG. 1

FIG. 2

FIG. 3

FIG. 4A

FIG. 4B

FIG. 5

FIG. 6

FIG. 7

FIG. 8

FIG. 9

FIG. 10

Europäisches
Patentamt

European
Patent Office

Office européen
des brevets

# EUROPEAN SEARCH REPORT

**Application Number**

EP 24 17 6213

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2008/009771 A1 (PERRY JOEL [US] ET AL) 10 January 2008 (2008-01-10) * paragraph [0248] - paragraph [0251]; figure 1 * * the whole document * | 4,5,11, 15 | INV. A61H1/02 |
| Y | US 2007/066918 A1 (DEWALD JULIUS P [US] ET AL) 22 March 2007 (2007-03-22) * paragraph [0003] * * paragraph [0106] * * paragraph [0029] - paragraph [0031] * * paragraph [0133]; figures 1-19 * * the whole document * | 4,5,11, 15 | |
| Y | US 11 090 801 B2 (UNIV NORTHERN ARIZONA [US]) 17 August 2021 (2021-08-17) * figures 1-11 * * the whole document * | 4,5,11, 15 | |
| X | US 2007/191743 A1 (MCBEAN JOHN M [US] ET AL) 16 August 2007 (2007-08-16) | 1,3,6-14 | |
| Y | * paragraph [0051]; figures 1-5 * * paragraph [0030] * * paragraph [0073] * * paragraphs [0059] - [0068] * * the whole document * | 4,5,11, 15 | TECHNICAL FIELDS SEARCHED (IPC) A61H A61B |
| Y | US 8 359 123 B2 (UNIV HONG KONG POLYTECHNIC [CN]; TONG KAIYU [CN]; SONG RONG [CN]) 22 January 2013 (2013-01-22) * the whole document * | 4,5,11, 15 | |
| X | US 2020/030177 A1 (ZENTGRAF JOHN [US] ET AL) 30 January 2020 (2020-01-30) | 1-3,6-9 | |
| Y | * the whole document * * paragraph [0084] - paragraph [0086] * * paragraph [0090] * * paragraph [0019] * * paragraph [0058] - paragraph [0059] * | 4,5 | |

The present search report has been drawn up for all claims

| Place of search | Date of completion of the search | Examiner |
|---|---|---|
| Munich | 25 September 2024 | Schindler-Bauer, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 6213

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2024

| Patent document cited in search report | | Publication date | Patent family member(s) | | Publication date |
|---|---|---|---|---|---|
| US 2008009771 | A1 | 10-01-2008 | US 2008009771 A1 | | 10-01-2008 |
| | | | US 2012179075 A1 | | 12-07-2012 |
| US 2007066918 | A1 | 22-03-2007 | CA 2581587 A1 | | 13-04-2006 |
| | | | US 2006079817 A1 | | 13-04-2006 |
| | | | US 2007066918 A1 | | 22-03-2007 |
| | | | WO 2006039403 A1 | | 13-04-2006 |
| US 11090801 | B2 | 17-08-2021 | US 2019344434 A1 | | 14-11-2019 |
| | | | US 2021339381 A1 | | 04-11-2021 |
| US 2007191743 | A1 | 16-08-2007 | US 2004106881 A1 | | 03-06-2004 |
| | | | US 2007191743 A1 | | 16-08-2007 |
| US 8359123 | B2 | 22-01-2013 | CN 101061984 A | | 31-10-2007 |
| | | | US 2009259338 A1 | | 15-10-2009 |
| | | | WO 2007128225 A1 | | 15-11-2007 |
| US 2020030177 | A1 | 30-01-2020 | CN 110997245 A | | 10-04-2020 |
| | | | JP 2020509834 A | | 02-04-2020 |
| | | | US 2020030177 A1 | | 30-01-2020 |
| | | | US 2020179213 A1 | | 11-06-2020 |
| | | | WO 2018165413 A1 | | 13-09-2018 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **J. WAGNER ; T. SOLIS-ESCALANTE ; P. GRIESHOFER ; C. NEUPER ; G. MULLER-PUTZ ; R. SCHERER.** Level of participation in robotic-assisted treadmill walking modulates midline sensorimotor eeg rhythms in able-bodied subjects. *Neuroimage,* 2012, vol. 63 (3), 1203-1211

- **G. TACCHINO ; M. GANDOLLA ; S. COELLI ; R. BARBIERI ; A. PEDROCCHI ; A. M. BIANCHI.** Eeg analysis during active and assisted repetitive movements: evidence for differences in neural engagement. *IEEE Transactions on Neural Systems and Rehabilitation Engineering,* 2016, vol. 25 (6), 761-771

- Detection of intention level in response to task difficulty from eeg signals. **E. KOYAS ; E. HOCAOGLU ; V. PATOGLU ; M. CETIN.** 2013 IEEE International Workshop on Machine Learning for Signal Processing (MLSP). IEEE, 2013, 1-6

- **S. PAREEK ; H. MANJUNATH ; E. T. ESFAHANI ; T. KESAVADAS.** Myotrack: Realtime estimation of subject participation in robotic rehabilitation using semg and imu. *IEEE Access,* 2019, vol. 7, 76 030-76 041

- **K. LANGLOIS ; E. ROELS ; G. VAN DE VELDE ; C. ESPADINHA ; C. VAN VLERKEN ; T. VERSTRATEN ; B. VANDERBORGHT ; D. LEFEBER.** Integration of 3d printed flexible pressure sensors into physical interfaces for wearable robots. *Sensors,* 2021, vol. 21 (6), 2157

- **K. LANGLOIS ; J. GEEROMS ; G. VAN DE VELDE ; C. RODRIGUEZ-GUERRERO ; T. VERSTRATEN ; B. VANDERBORGHT ; D. LEFEBER.** Improved motion classification with an integrated multimodal exoskeleton interface. *Frontiers in Neurorobotics,* 2021, 140

- **F. LEONE ; C. GENTILE ; F. CORDELLA ; E. GRUPPIONI ; E. GUGLIELMELLI ; L. ZOLLO.** A parallel classification strategy to simultaneous control elbow, wrist, and hand movements. *Journal of NeuroEngineering and Rehabilitation,* 2022, vol. 19 (1), 1-17

- **B. ZADROZNY ; C. ELKAN.** Transforming classifier scores into accurate multiclass probability estimates. *Proceedings of the eighth ACM SIGKDD international conference on Knowledge discovery and data mining,* 2002, 694-699

- **A. NICULESCU-MIZIL ; R. CARUANA.** Predicting good probabilities with supervised learning. *Proceedings of the 22nd international conference on Machine learning,* 2005, 625-632

- **T.-M. BYNION ; M. T. FELDNER.** Self-Assessment Manikin. Cham. Springer International Publishing, 2017, 1-3

- **L. M. A. LA BARA ; L. MELONI ; D. GIUSINO ; L. PIETRANTONI.** Assessment methods of usability and cognitive workload of rehabilitative exoskeletons: A systematic review. *Applied Sciences,* 2021, vol. 11 (15), 7146

- **SHARKAWY et al.** Human-Robot Interaction : A review and Analysis on Variable Admittance Control, Safety, and Perspectives. *Machines,* 2022, vol. 10 (7), 1-26

- **LYNCH et al.** Video Supplements for Modern Robotics. Cambridge University Press

- **LANGLOIS K. ; RODRIGUES CIANCA D ; ERRIEN, B ; DE WINTER, J ; VERSTRATEN, T. ; RODRIGUEZ GUERRERO, C. D ; VANDERBORGHT, B. ; LEFEBER, D.** Investigating the effects of strapping pressure on human-robot interface dynamics using a soft robotic cuff. *IEEE transactions on medical robotics and bionics.,* 2020, vol. 3 (1), 146-155